Europäisches Patentamt

European Patent Office

Office européen des brevets

(19)

(11) Publication number: **0 314 496**
A2

# (12) EUROPEAN PATENT APPLICATION

(21) Application number: **88310154.5**

(22) Date of filing: **28.10.88**

(51) Int. Cl.4: **C 12 N 5/00**
**C 12 P 21/00**

(30) Priority: **29.10.87 US 114054**

(43) Date of publication of application:
**03.05.89 Bulletin 89/18**

(84) Designated Contracting States:
**BE CH DE FR GB IT LI NL SE**

(71) Applicant: **E.I. DU PONT DE NEMOURS AND COMPANY**
**1007 Market Street**
**Wilmington Delaware 19898 (US)**

(72) Inventor: **Largen, Michael Terry**
**11 Quartzmill Road**
**Newark Delaware 19711 (US)**

(74) Representative: **Myerscough, Philip Boyd et al**
**J.A.Kemp & Co. 14, South Square Gray's Inn**
**London, WC1R 5EU (GB)**

(54) **Improved hybridoma production.**

(57) Low protein content, serum-free media, containing hybridoma growth factors, are provided for growth of hybridoma cell lines and antibody production.

EP 0 314 496 A2

Bundesdruckerei Berlin

## Description

## IMPROVED HYBRIDOMA PRODUCTION

### FIELD OF THE INVENTION

This invention relates to the use of growth factors contained in serum-free conditioned medium from primary cultures or immortal lines of various cells for the serum-free growth and in vitro production of monoclonal antibodies by B cell hybridomas.

### BACKGROUND ART

In 1975 Kohler and Milstein [Nature 256:495-497] described the fusion of myeloma cells and immune spleen cells to produce immortal cells secreting antibodies of predetermined specificities. These cell lines, called hybridomas, each produce a single homogeneous antibody which is the product of a single immortalized B cell clone. Previously only polyclonal antisera derived from in vivo immunization of whole animals could be obtained. Polyclonal antisera contain heterogeneous mixtures of antibodies produced, in most cases, from many different B cell clones. A review article by Milstein [Scientific American 243:66-74, 1980] summarizes the advantages of monoclonal antibodies as compared to the conventional polyclonal antibodies raised in experimental animals.

Cell lines producing monoclonal antibodies can be grown in vivo by injecting the cell line intraperitoneally to produce ascites tumors. The ascites fluid is a rich source of monoclonal antibody (1-10 mg/mL usually) but, unfortunately, also contains high concentrations of non-specific polyclonal immunoglobulin molecules as well as other proteins. The alternative method of monoclonal antibody production is in vitro culture of the hybridoma cell line. The use of serum-containing culture medium (typically 10-20% fetal calf serum) is usually required for cell growth and antibody production. However, the use of serum in the medium gives high concentrations of serum proteins which must be removed from the immunoglobulin of interest. Various formulations of media which allow the growth of B cell hybridoma cell lines in the absence of serum (serum-free growth) have been described [Murakami et al., Proc. Natl. Acad. Sci. USA 79:1158-1162 (1982) and Darfler et al., Exp. Cell Res. 138:287-295 (1982)] and are available commercially.

The various formulations for serum-free culture media for hybridomas contain other growth supporting components such as insulin, transferrin, ethanolamine, and fatty acid-linked BSA (see references above). The protein concentration of these media are often quite high, e.g. 750 μg/mL protein. Such a high protein concentration, although much lower than that of the medium supplemented with 10% fetal calf serum (FCS), still poses a purification problem.

The purpose of using serum-free medium to grow hybridoma cells for antibody production is to reduce the protein concentration of the medium to as low a level as possible in order to simplify the purification of antibodies. Different medium formulations are often required for growth of hybridoma cells originally made using different myeloma cell lines. The currently available serum-free medium formulations that will grow a random selection of hybridoma cell lines have high protein content. In contrast, those serum-free medium formulations which have low levels of protein (not more than 50 μg/mL) do not support the growth of a high percentage of a random sample of hybridoma cell lines.

In addition, currently available serum-free medium formulations for hybridoma cell lines, whether they contain high or low protein levels, require stepwise adaptation of cells from serum-containing media to serum-free media. This stepwise adaptation involves sequential passages of the cells through media which contains increasingly higher concentrations of serum-free versus serum-containing media. This adaptation is time consuming, requiring up to six weeks, and is sometimes unsuccessful for particular hybridoma cell lines. Stepwise adaptation is especially required for serum-free medium formulations with low protein content.

The use of feeder cells (e.g. endothelial cells, peritoneal macrophages, spleen cells) to improve the plating efficiency of fusion mixtures and to increase the cloning efficiency of hybridoma cell lines in serum-containing media has been described [Lernhardt et al., Exp Cell Res. 111:309 (1978) and Fazekas de St. Groth et al., J. Immunol. Meth. 35:1 (1980)]. One of the properties of feeder cells is to produce and secrete soluble growth factors. These factor(s) can be obtained in crude form from so-called conditioned media. Conditioned medium is medium in which these feeder cells have been grown and from which the cells have been subsequently removed. Conditioned media from endothelial [Astaldi et al., J. Immunol. 125:1411 (1980)] and monocyte/macrophage cells [Sugasawara et al., J. Immunol. Meth 79:263-275 (1985) and Rathjen et al., Hybridoma 5:255-261 (1986)] have been found to be useful in this regard.

German Patent Application DE 3,523,202 A 1 describes the use of cell-free, serum-containing (5-10% fetal calf serum) media conditioned with suitable cells such as mouse spleen or lymph node cells, or thymocytes to supplement serum-containing media to enhance the cloning efficiency and expansion of hybridomas, particularly human hybridomas. The presence in the disclosed serum-containing media of high levels of protein subjects these media to the same disadvantages described above.

The beneficial effects of conditioned medium on hybridoma cell growth are the results of growth factors (proteins) secreted by the feeder cells. A secreted protein from mouse T lymphocytes, termed hybridoma growth factor (HGF), has been purified and the amino-terminus amino acid sequence determined [Van Snick et al., Proc. Natl. Acad. Sci. USA 83:9679, 1986]. This protein has been shown to stimulate the growth of certain hybridomas in serum-containing medium. A protein which also stimulates the in vitro growth of myelomas (B cell tumors which are used as one of the parents in the production of B cell hybridomas) has been purified from a continuous monocyte/macrophage cell line [Nordan et al., J. Immunol. 139:813, 1987]. The amino terminus sequence of this macrophage-derived growth factor is similar to, but not identical with, the T cell factor. In addition, human interferon beta-2 has been shown to be identical to human B cell stimulating factor-2 (BSF-2) and to have growth factor activity for hybridomas and myelomas in serum-containing medium [Van Damme et al., J. Exp. Med. 165:914, 1987].

To date, the use of growth factors, either in purified form or in serum-free conditioned medium, to support the serum-free growth of B cell hybridomas has not been described.

## SUMMARY OF THE INVENTION

The invention described herein utilizes growth factors as a component of low protein-containing serum-free medium for growth of B cell hybridoma cells and in vitro production of monoclonal antibodies without any prior adaptation of the hybridoma cell lines to serum-free media.

The growth factors can be provided in purified form from natural sources, as a recombinant protein or in the form of a serum-free conditioned medium.

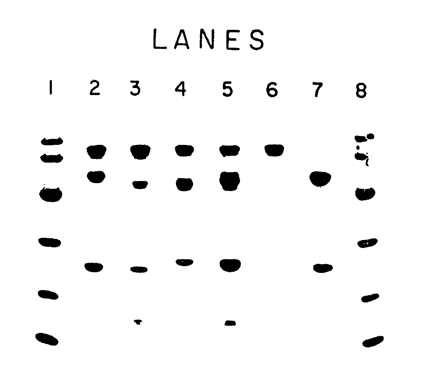

## BRIEF DESCRIPTION OF THE DRAWING

The Figure is a photograph of the gel electrophoretogram of antibodies obtained from hybridomas grown in the media of this invention.

## DESCRIPTION OF THE INVENTION

The improved low protein content, serum-free medium of this invention is useful for growing B cell hybridoma cell lines through the unexpected finding that in presence of a growth factor the cell lines did not need to be adapted to low protein, serum-free media.

The growth factor to be utilized can be in purified form obtained from natural sources such as by purification of naturally occurring hybridoma growth factors. The growth factor can also be recombinant DNA-produced protein or it can also be supplied in the form of a conditioned, serum-free medium. By conditioned medium is meant a medium containing growth factor but not containing the cells producing the growth factors.

The conditioned, serum-free medium preferably utilized in this invention, to afford the improved low protein content, serum-free medium useful for hybridoma growth, can be prepared by incubating feeder cells in a serum-free medium with a suitable inducing agent (when such is necessary), followed by removing the cells and cellular debris by centrifugation and/or (sterile) filtration. The incubation of the feeder cells can be carried out in low protein, serum-free media, many of which are commercially available, for 24-48 hours in presence of suitable stimulating ("inducing") agents. Such stimulating agents, when necessary, can stimulate the feeder cells to produce and secrete growth factors.

A preferred inducing agent for monocyte/macrophage cell lines is bacterial lipopolysaccharide (LPS) extracted from the cell wall of Gram negative bacteria. (Monocyte/macrophage cell lines are cell lines which have monocyte/macrophage characteristics such as having murine Mac-1, Mac-2 or Mac-3 cell surface markers). Other inducing agents such as phorbol myristate acetate or other phorbol esters and muramyl dipeptide (MDP) can also be effective for monocyte/macrophage cell lines. The effectiveness of other inducing agents for monocyte/macrophages, as well as other cell types, can be ascertained using the assay described in the Example.

Monocyte/macrophage cells lines are preferred feeder cells for the production of growth factors useful in this invention but other cells such as endothelial or spleen cells can also be used provided they are themselves able to grow and produce growth factors in low protein content, serum-free media.

The ability of various cells lines or primary cultures to produce growth factors is readily assayed by the practical expedient of incubating the cells in low protein, serum-free medium and then determining the ability of this medium, when used as a supplement, to promote the growth of hybridoma cells in low protein, serum-free media. If hybridoma cell growth is promoted, the cell lines can be utilized for the preparation of the conditioned, serum-free media useful for preparing the improved media of this invention.

Once prepared, the conditioned medium can be added as a supplement to basal, low protein, serum-free media to which insulin, transferrin, and ethanolamine can also be added. Most murine hybridoma cell lines customarily maintained in serum-containing medium can be transferred directly to the above prepared medium of this invention (without prior adaptation of the hybridoma to low protein, serum-free medium) where the cell lines will grow and produce amounts of immunoglobulin (antibodies) comparable to those produced in conventional serum-containing media. The medium of this invention is useful for growing cell lines capable of producing either IgG or IgM antibodies.

The low-protein content improved media of this invention contain approximately 50 µg/mL protein or less and are customarily prepared by the addition of various amounts of the conditioned medium, but preferably approximately 5% (volume/volume) or less, to the modified basal serum-free medium ( which is low in protein content). Many of these low protein content media are available commercially; for example, Nutridoma (Boehringer Mannheim), HB301 (Hana Biologics), HL-1 (Ventrex), and GIT (ICN Biochemicals). The basal medium can be modified with a variety of chemicals such as insulin, transferrin, selenous acid, ethanolamine and fatty acids. A very effective serum-free formulation useful in the instant invention is 50% DME (Dulbecco's Modified Eagles Medium) and 50% F-12 (Hank's F-12 Medium), to provide basal salts and nutrients, supplemented with 2.4 mM L-glutamine, 5 µg/mL bovine insulin, 30 µg/mL human transferrin, sodium selenite 2.6 ng/mL, 20 µM ethanolamine, and 0.5% (v/v) conditioned medium.

The Example below illustrates the invention.

## EXAMPLE

### In Vitro Preparation of Monoclonal Antibodies

#### A. Assay for Hybridoma Growth Factor Production

In order to identify appropriate cell lines useful in preparing conditioned medium as well as inducing agents necessary in certain instances for its production, it was necessary to establish a quantitative assay for detection of the growth factor(s) in conditioned medium capable of providing for non-adapted growth of hybridoma cells in low protein content serum-free medium. A reproducible, sensitive, semi-automated assay was devised which makes it possible to assay large numbers of samples suspected of containing hybridoma growth factors for hybridoma growth promoting activity in serum-free media. First a hybridoma cell line was plated at low density in a non-protein containing basal tissue culture medium supplemented with a low protein content serum substitute. Samples of conditioned medium containing growth factors were then added and after incubation for approximately 48-72 hours, proliferation and/or activation was measured as described below and compared to control cells to which no conditioned medium had been added.

For the preparation of conditioned medium, RAW 264.7 (ATCC TIB 71) cells were first adapted to growth in low protein serum-free medium (SM) containing 50% DME (Dulbecco's Modified Eagle's Medium, Hazelton-Dutchland) and 50% F-12 (Hank's F-12 Medium, Hazelton-Dutchland) supplemented with 2.4 mM L-glutamine, 5 µg/mL bovine insulin (Sigma Chemicals), 30 µg/mL human transferrin (Sigma), and sodium selenite, 2.6 ng/mL. Cells were adapted to growth in SM by growing for several passages in such medium at which time growth rate was approximately 80-90% of that obtained in medium containing 10% FCS. Cells were grown to near confluence (7-10 x $10^5$ cells/mL) and LPS (E. coli 055:B5W, Difco) was added to a final concentration of 0.1 Mg/mL. After incubation for 48 hours, supernatant was removed and cells and cellular debris were removed by centrifugation at 48,000 xg for 15 minutes. Additional smaller debris was removed and the solution rendered sterile by passage through sterile 0.22 micron filters. This conditioned medium was frozen in aliquots at -70°C or could be stored for several weeks at 4°C.

The assay utilized was substantially similar to the tetrazolium dye procedure of Denizot et al. [J. Immunol. Meth. 89: 271-279 (1986)]. Cells for testing for growth [hybridoma line 41I/47.2 produced by a standard Kohler and Milstein protocol utilizing FSH (follicle stimulating hormone) as the immunogen] were plated as indicated in Table 1 and grown in 96 well plate whole area cells (Costar Scientific) for 48 hr in a humidified $CO_2$ incubator at 37°C. The medium used was Iscove's medium supplemented with 1% Nutridoma SP (Boehringer-Mannheim) with conditioned medium added as shown in Table 1. Just prior to the assay, the medium was removed with an 8-channel vacuum manifold. Then, 100 µl of MTT [3-(4,5-dimethylthiazol-2-yl)-2,5-diphenyl tetrazolium bromide] (Sigma Chemical Co., 1 mg/mL in Iscove's medium) was added to each well. The 96 well plates were incubated at 37°C in a humidified $CO_2$ incubator for an additional 3 hr with occasional gentle shaking. At the end of the incubation period, the 96 well plates were centrifuged for 5 min at 800 x g and the medium was aspirated from the wells with an 8-channel manifold. One hundred µL of 100% isopropanol containing 0.04 N HCl was added and the plates were shaken vigorously for 60 seconds to solubilize the formazan dye produced from MTT by the viable cells. The optical density of the dye solutions in the plate wells was measured on an automatic plate reader at 570 nm test wavelength and 690 nm reference wavelength and the data summarized in Table 1.

TABLE 1

| CONDITIONED MEDIUM (µL) | CELLS/WELL | MTT CONVERTED (O.D., FORMAZAN SOLUTION) |
|---|---|---|
| 0 | 1250 | .018 |
| 10 | " | .023 |
| 20 | " | .028 |
| 30 | " | .043 |
| 40 | " | .041 |
| 50 | " | .041 |
| 100 | " | .038 |
| 0 | 2500 | .033 |
| 10 | " | .057 |
| 20 | " | .063 |
| 30 | " | .080 |
| 40 | " | .077 |
| 50 | " | .077 |
| 100 | " | .077 |
| 0 | 5000 | .035 |
| 10 | " | .091 |
| 20 | " | .105 |
| 30 | " | .131 |
| 40 | " | .139 |
| 50 | " | .139 |
| 100 | " | .139 |

The results in Table 1 show that increasing the amount of conditioned medium, up to approximately 30-40 µL, increases hybridoma growth resulting in increased MTT conversion. MTT conversion to a formazan dye is proportional to the number of cells present in each well. When the same concentration of conditioned medium was added to more target cells, there was an approximately proportional increase in hybridoma growth.

Similar results were obtained using alkaline phosphatase (instead of MTT) as an indicator of B cell proliferation following the protocol of Hasimoto et al. [J. Immunol. Meth. 90:97-103 (1986)]. The results were similar to those obtained with the MTT assay, although the alkaline phosphatase assays were somewhat less sensitive.

B. Derivation of Cell Lines Producing Hybridoma Growth Factors

Cell lines with monocyte/macrophage characteristics, including production of hybridoma growth factors, were derived from long term murine spleen cell cultures. Spleens from Balb/c mice were surgically removed and passed through a small mesh screen to prepare a single cell suspension. Each spleen was placed in a T-75 flask containing 20 mL of Iscove's medium containing hypoxanthine (16.3 mg/mL), thymidine (9.12 mg/mL), glutamine (2.4 mM), 2-mercaptoethanol(5 x $10^{-5}$M), and 10%(v/v) FCS. After incubating for approximately 1 day, flasks were rocked gently to remove most erythrocytes and other non-adherent cells, followed by the removal of the medium by aspiration and its replacement with 20-25 mL fresh medium. At day 5-7, day 14-18, and day 24-28, 10 mL medium was removed and fresh medium was added. Between days 30 and 60, depending upon the individual spleen cell culture, rapidly dividing cells were observed. Ten to 14 days later cell lines were cloned by limiting dilution in 96 well plates. A large number of clones which grew immortally in culture without the addition of exogenous growth factors and which were readily adaptable to growth in SM were obtained. These cells expressed surface antigens such as Mac-1, Mac-2, and Mac-3 which are characteristic of monocyte/macrophage lineage.

A series of clones, designated SPL-4.1, 4.2, 4.3, and 4.4, obtained by culture of limiting dilutions of one of these original cell lines, SPL-4 were grown in SM for 48 hr in the presence of 0.1 µg/mL LPS (S.typhimurium Re 595, RIBI), centrifuged, and the resultant conditioned media were assayed by the MTT assay (8000 24E/3 cells/wells) for 48 hr to test for the production of hybridoma growth factors. The data are summarized in Table 2.

TABLE 2

| AMOUNT OF CONDITIONED MEDIA (µL) | MTT CONVERTED (O.D. 570 nm) | | | |
|---|---|---|---|---|
| | SPL 4.1 | SPL 4.2 | SPL 4.3 | SPL 4.4 |
| 0 | .014 | .018 | .015 | .009 |
| 5 | .073 | .024 | .103 | .049 |
| 10 | .102 | .024 | .100 | .097 |
| 20 | .080 | .031 | .105 | .088 |

As can be seen for Table 2, all of these cells lines, except for SPL 4.2, produced growth factors capable of stimulating hybridoma growth in the medium of this invention.

C. Production of Monoclonal Antibodies in Low Protein-Containing Serum-Free Medium

The hybridoma cell lines for producing IgG's of IgM's utilized in this Example were obtained through standard Kohler and Milstein protocols utilizing the indicated immunogens:

| HYBRIDOMA | IMMUNOGEN |
|---|---|
| 24E/3 | $N^5$-methyltetrahydrofolate-KLH |
| 24E/8 | $N^5$-methyltetrahydrofolate-KLH |
| 24E/10 | $N^5$-methyltetrahydrofolate-KLH |
| 36I/13.1.2 | luteinizing hormone (LH) |
| 85J/34 | phenytoin-KLH |
| 29A/46.1 | β-galactosidase (from E. coli) |
| 46/67 | 2-chloro-N-[(4-methoxy-6-methyl-1,3,5-triazin-2-yl)aminocarbonyl]benzenesulfonamide-BSA |

The amount of IgG secreted by various hybridoma clones selected at random was compared in three different media: low protein-content serum-free medium (Iscove's basal medium plus Nutridoma), the same basal medium supplemented with 10% FCS, and the medium of this invention (Iscove's plus Nutridoma plus conditioned medium). The amounts of IgG secreted by cultures grown to confluence were measured in an ELISA assay which was calibrated using myeloma protein MOPC 141 (Litton Bionetics).

Several hybridoma cell lines were grown in the three different media described above. Cells were inoculated at $5 \times 10^4$ cells/mL and, following 4-7 days growth to confluence, the IgG content of the supernatant was determined for each of the media in the ELISA as described below.

For the ELISA assay, Immulon II 96 well plates (Dynatech) were washed with distilled water and then coated with 100 µL/well of a 50 µg/mL solution of rabbit anti-mouse IgG (gamma chain-specific, Zymed Laboratories) which was made up in PBS, pH 7.4 (Sigma). After overnight coating at 4°C, the plates were washed 5x with wash buffer (0.15M NaCl, 0.01M $NaH_2PO_4$, pH 7.8) and blotted dry. Each well was blocked with 200 µL/well of 3%(w/v) bovine serum albumin (BSA) in wash buffer. The plates were again washed 5x with wash buffer and blotted dry. The hybridoma culture supernatants to be assayed for IgG were titered in 2-fold dilutions across the plate by adding 50 µL of each dilution. The plate was covered with sheets of Parafilm wax and incubated at 37°C for 1 hour. The plate was then washed 6x with wash buffer, pH 7.8. One hundred µL of biotinylated rabbit anti-mouse IgG (Gamma chain-specific, Zymed Laboratories), diluted 1:400 in wash buffer, pH 7.8, containing 1%(w/v) BSA, was added to each well and the covered plate was incubated for 1 hour at room temperature. The plate was then washed 6x in wash buffer, pH 7.8, and 100 µL/well of a streptavidin-alkaline phosphatase conjugate (Zymed Laboratories) diluted 1:1000 in wash buffer, pH 7.8 (containing 1% BSA) was added to each well. Following incubation for 1 hour at room temperature, plates were washed 12x with wash buffer pH 7.8. One hundred µg of p-nitrophenyl phosphate (PNPP) dissolved in substrate buffer (0.1M diethanolamine, pH 10.3) was added to each well and incubated for 20 minutes at room temperature at which time the optical density at 405 nm was read in an automated plate reader (MR600, Dynatech). The optical density values were compared with a standard curve using MOPC 141 IgG myeloma protein (Litton Bionetics) and the IgG concentration for each cell line/medium was determined by extrapolation to the standard curve. The data are summarized in Table 3 below.

TABLE 3

IgG PRODUCTION IN VARIOUS MEDIA

| CELL LINE | MEDIUM | IgG CONCENTRA-TION ($\mu$g/mL) |
|---|---|---|
| 411/47.2 | basal + 1% Nutridoma | 2.5 |
| " | basal + 10% FCS | 60 |
| " | basal + 1% Nutridoma + 5% RAW 264.7[1] | 40 |
| " | basal + 1% Nutridoma + 5% SPL-4[2] | 40 |
| 24E/8 | basal (only) | 1 |
| " | basal + 1% Nutridoma | 2.5 |
| " | basal + 10% FCS | 50 |
| " | basal + 1% Nutridoma + 5% RAW 264.7 | 55 |
| " | basal + 1% Nutridoma + 5% SPL-4 | 55 |
| 24E/10 | basal + 1% Nutridoma | 5 |
| " | basal + 10% FCS | 45 |
| " | basal + 1% Nutridoma + 5% RAW 264.7 | 10 |
| " | basal + 1% Nutridoma + 5% SPL-4 | 50 |

(1) conditioned medium (v/v) containing growth factors produced by RAW 264.7.
(2) conditioned medium (v/v) containing growth factors produced by SPL-4.

Several other cell lines were tested under these same growth conditions and most responded to growth factors present in the monocyte/macrophage CM by growth and IgG or IgM production. In all but one case (24E/10 + 1% Nutridoma + 5% RAW 264.7) the IgG or IgM levels were roughly comparable to those obtained with medium containing 10% FCS. Even in the 24E/10 + growth factor (RAW 246.7) case, the IgG content was twice that found without the growth factor. A variety of commercially available low protein content, serum-free media formulations were also tested for their ability to promote hybridoma growth when in presence of the monocyte/macrophage-generated growth factors. Satisfactory IgG production was observed and in no case did this require preadaptation of the cell lines, a step universally recommended by manufacturers for the low protein, serum-free formulations. In general, serum-free, low protein media without growth factors did not promote hybridoma growth. (It should be noted that in a few instances the inventor has observed some cell lines, especially those that have been passaged for a long time in vitro, which required no preadaptation and which grew and produced high levels of IgG in low protein, serum-free medium without the addition of growth factor.) Cell lines grown for multiple passages in the low protein, serum-free medium of this invention continue to grow and produce antibody at high concentrations.

The data in Table 3 show that approximately equal amounts of IgG produced in serum-containing, high protein-content media of the prior art and in the low protein content, serum-free media of this invention.

Additionally, determination of antigen specific titers by ELISA of the IgG's produced were found to be in broad agreement with IgG concentrations shown above leading to the conclusion that the antibodies produced under the various growth conditions were of similar functional ability with respect to antigen binding.

### D. Purity of IgG Produced Under Various Growth Conditions

Various hybridoma lines were grown to confluence using a low protein, serum-free medium of this invention [SM containing 20 µM ethanolamine and 0.5% (v/v) LPS-induced (0.1 mg/mL S. minnesota Re 595, Ribi Immunochemicals) supernatant from clone SPL 4.3]. The medium was removed and centrifuged, the cell-free supernatants were concentrated 50X using a Minicon disposable concentration device (Amicon), and samples were diluted 1:4 or 1:8 with SDS sample buffer (final concentration 0.625M Tris-HCl, pH 6.8, 1.05% SDS, 10% glycerol, and 5% 2-mercaptoethanol). One-µl samples were applied to a 10-15% SDS PAGE gradient gels (Pharmacia) and run using the Pharmacia Phast System. Following electrophoresis, the gel was stained using Coomassie Brilliant Blue R250 also using the Phast gel system. The dried gel was photographed using the Kodak EDP system for visualization of Coomassie Blue-stained gels.

In the Figure, Lanes 1 and 8 contain low molecular weight markers which are, from top to bottom, phosphorylase b, 94,000 daltons; bovine serum albumin, 67,000 daltons; ovalbumin, 45,000 daltons; carbonic anhydrase, 30,000 daltons; soybean trypsin inhibitor, 20,100 daltons; and lactalbumin, 14,400 daltons. Lanes 2, 3, 4, and 5 contain 50x concentrates of 1:4 dilution of cell line 36I/13.1.2 supernatant, 1:4 dilution of cell line 85J/34 supernatant, 1:4 dilution of cell line 29A/46.1 supernatant, and 1:8 dilution of cell line 46/67 supernatant, respectively. Lane 6 contains SM supplemented with ethanolamine and SPL 4.3 supernatant (0.5% v/v) concentrated 50x and diluted 1:4. Lane 7 contains IgG(16/310.2) purified from ascites (concentration 0.2 mg/mL) using a protein A column.

As can be seen in the Figure, the purity of the IgG's produced from hybridomas grown in the medium of this invention is excellent. Only transferrin from the synthetic medium (cf. Lane 6) is present in appreciable concentration in addition to the IgG (see the heavy and light chains of IgG: cf. Lane 7). Although not shown, when FCS was used, there were a multitude of bands present with bovine serum albumin as the dominant component. In electrophoretograms of supernatants from cells grown in medium containing 10% FCS, IgG could not even be identified.

### Claims

1. A low protein content, serum-free medium for hybridoma growth, which medium contains a growth factor.

2. A medium according to claim 1 wherein the growth factor is provided in the form of a serum-free conditioned medium.

3. A medium according to claim 1 wherein the growth factor is provided in a purified form

4. A medium according to claim 1, 2 or 3 wherein the growth factor has been produced by a cell line with monocyte/macrophage characteristics.

5. A medium according to claim 1 wherein the growth factor is a recombinant protein.

6. A medium according to any one of the preceding claims wherein the protein content is not more than 50 µg/mL/

7. Use of a serum-free medium as claimed in any one of the preceding claims in the cultivation of a hydridoma cell line producing a monoclonal antibody.

LANES

1  2  3  4  5  6  7  8